# EUROPEAN PATENT SPECIFICATION

(11) **EP 3 991 725 B1**
(45) Date of publication and mention of the grant of the patent: **28.01.2026**
(21) Application number: 20020504.5
(22) Date of filing: 02.11.2020
(51) Int. Cl.: A61K 31/05, A61K 9/00, A61P 31/04, A61P 31/00, A61K 31/165, A61K 9/20, A61K 9/28

(54) **NOVEL ENTERO-ANTISEPTIC FOR THE TREATMENT OF GASTROINTENSTINAL INFECTIONS IN HUMANS**
NEUES ENTEROANTISEPTIKUM ZUR BEHANDLUNG GASTROINTESTINALER INFEKTIONEN BEI MENSCHEN
NOUVEL ENTÉRO-ANTISEPTIQUE POUR LE TRAITEMENT DES INFECTIONS GASTRO-INTESTINALES CHEZ L'HOMME

(43) Date of publication of application: 04.05.2022
(73) Proprietor: Ninkov, Dusan, 1066 Budapest (HU)
(72) Inventor: Ninkov, Dusan, 1066 Budapest (HU)
(74) Representative: Filipova, Liljana

(56) References cited:
- WO-A1-2013/182709
- WO-A1-2020/208344
- WO-A1-97/01348

## Description

### FIELD OF THE INVENTION

The compound is a novel entero-antiseptic for the treatment of gastrointenstinal infections in humans.

### BACKGROUND OF THE INVENTION

This invention is addressing the problems of gastrointestinal infections in humans with the different etiology of pathogens. The compound is a broad-spectrum entero-antiseptic compound in the form of a 600 mg tablet for the treatment of acute infective gastroenteritis. The antimicrobial effect of Thymol and Carvacrol is well known. Several patents based on Carvacrol and Procaine-Carvacrol have been granted in the US and the EU patent offices. One of such patents is a US patent no. 6,921,539 and the same invention in the EU as an EU patent no. EP 1 482 791 B1. WO97/01348 discloses a pharmaceutical composition comprising both thymol and carvacrol for use as antimicrobial against i.a. salmonella, staphylococcus and E. coli. In particular, this invention is completely novel because Thymol and Carvacrol, by the special process of synthesis have been bounced into one stabile compound. The effect and uniqueness of this compound is the subject of this invention. The active principle of the compound is a stable quaternary ammonium complex of Thymol and Carvacrol in the form of a tablet. The compound is more effective than standard 3rd generation cephalosporin antibiotics in the treatment of mentioned infections. The compound is created to fight antibiotic resistance of pathogenic bacteria, such as E. *coli, Salmonella, Campylobacter, Shigella, Vibrio, Yersinia, Listeria,* and other bacteria causing acute infective gastroenteritis in humans. Based on the specific mechanism of action, the active compound of this invention is different from any other antibiotic currently available on the market. In this invention, an extra potent effect of the synergy of Thymol and Carvacrol is achieved. The antimicrobial efficacy of this novel compound will be significantly more effective than Thymol and Carvacrol by themselves or by their simple mixture.

Before the widespread use of Penicillin, infectious diseases were the leading cause of death among all age groups across the globe. Antibiotics have extended the lives and improved the quality of life of people since the early 20th century. Many have thought that humanity was entering the post-pathogen era, where infectious disease was a historical footnote. Today, infective diseases are still the first leading cause of death and the leading cause of disability.

### DESCRIPTION OF THE INVENTION

In the following text, the novelty and uniqueness of the compound will be elaborated.

### Method of production

The synthesis of our novel compound is carried out in a few steps via Steglich esterification. The first step was protecting the amino group of glycine (with a t-BOC protecting group) using a di-tert-butyl dicarbonate in the presence of 4-Dimethylaminopyridine (DMAP) as a base. Esterification of the carboxylic group of amino-protected glycine (Gly-NH-tBOC) with a phenol group of thymol is conducted using *N*,*N^{'}*-dicyclohexylcarbodiimide (DCC) as a carboxylic acid group activator and a 4-dimethylaminopyridine (DMAP) as a catalyst in dichloromethane (DCM) as solvent. We are watching the progress of our reaction by looking at the reaction samples periodically, using TLC plates under UV light, monitoring them for completeness.

After finishing the reaction ceases, we need to deprotect the t-BOC group from the amino group, preparing it for the next step. The t-BOC group was removed using trifluoroacetic acid (TFA). The next step is the basification of the reaction mixture using a saturated solution of sodium bicarbonate to obtain a free base of our product that is soluble in dichloromethane (DCM). After that, we extract our product using DCM and separate the water layer using a separation funnel. Now the free base ester of Thymol with Glycine is in the DCM layer, ready for reaction with Carvacrol. In the reaction of our obtained compound with Carvacrol, a novel compound the compound is formed as solution in DCM. After removing the solvent from the rotary vacuum evaporator, we measure the mass of the product and calculate the yield of the reaction.

### Schematic diagram of synthesis procedure

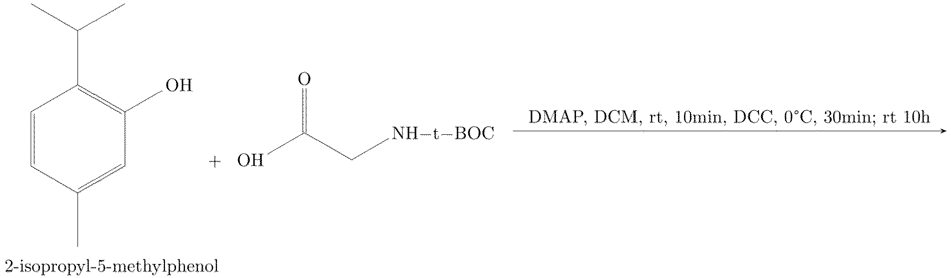

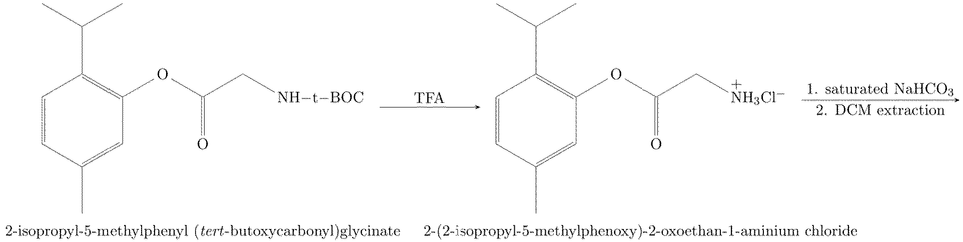

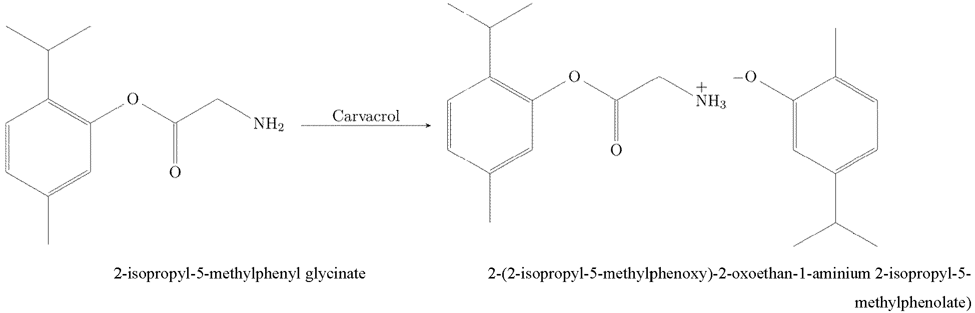

### Structural formula of the compound

Chemical formula: C₂₂H₃₁O₃N
Molecular mass: 357.501 g mol⁻¹
Compound chemical name:
   2-(2-isopropyl-5-methylphenoxy)-2-oxoethan-1-aminium 2-isopropyl-5-methylphenolate

### Characterization

The characterization of purity of our novel compound will be done using gass-mass chromatography, IR, 1H NMR and 13C NMR spectra. The position of the peaks in gass chromatography will provide us with data about the purity of the sample. The mass spectrometry will provide mass fragments specific for a certain compound. 1H NMR spectra will give us the position of protons in the molecule and we can compare its peaks with the peaks of the same positions of protons in the precursor compounds spectra as well as the spectra of pure Thymol and Carvacrol. The density of the compound is 1.097g mL⁻¹ and melting point is 68°C.

### Brief description of the Figures

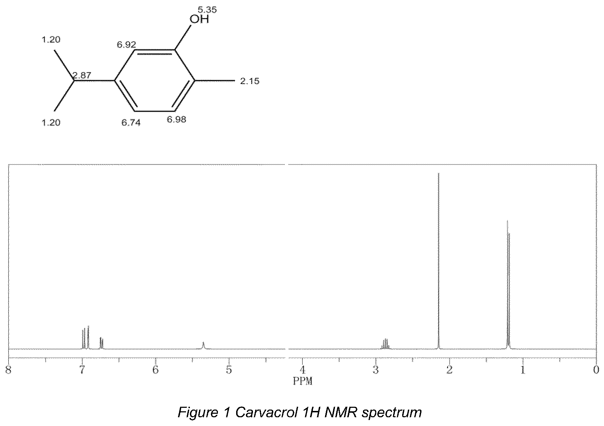

*Figure 1**: Carvacrol 1H NMR spectrum analysis, obtained from a reputable chemical database (http:*//*www.molbase.com*/*moldata*/*26822-spectrum.html)*

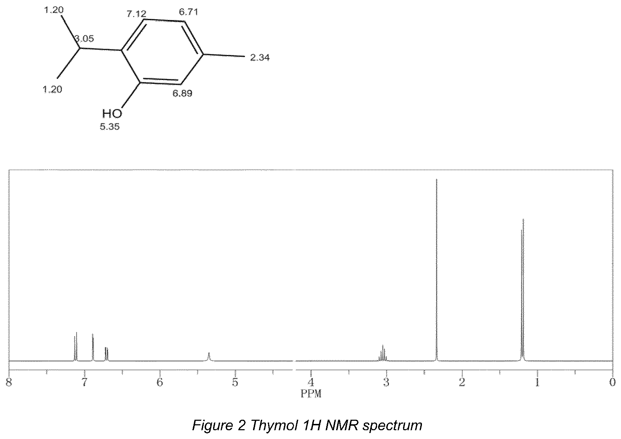

*Figure 2**: Thymol 1H NMR spectrum analysis, obtained from a reputable chemical database (http:*//*www.molbase.com*/*moldata*/*37232-spectrum.html)*

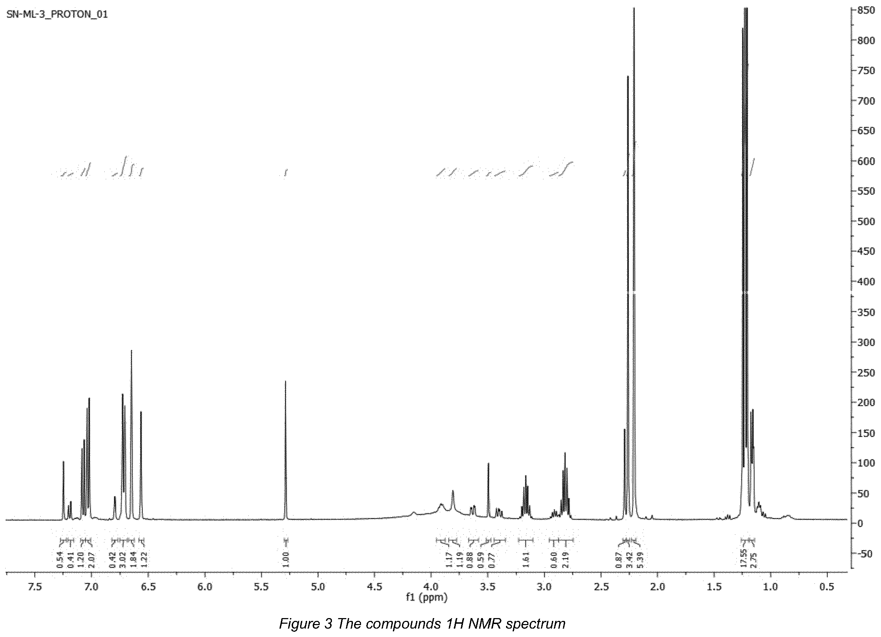

*Figure 3**: The compounds 1H NMR spectrum analysis, obtained after the synthesis of our novel compound.*

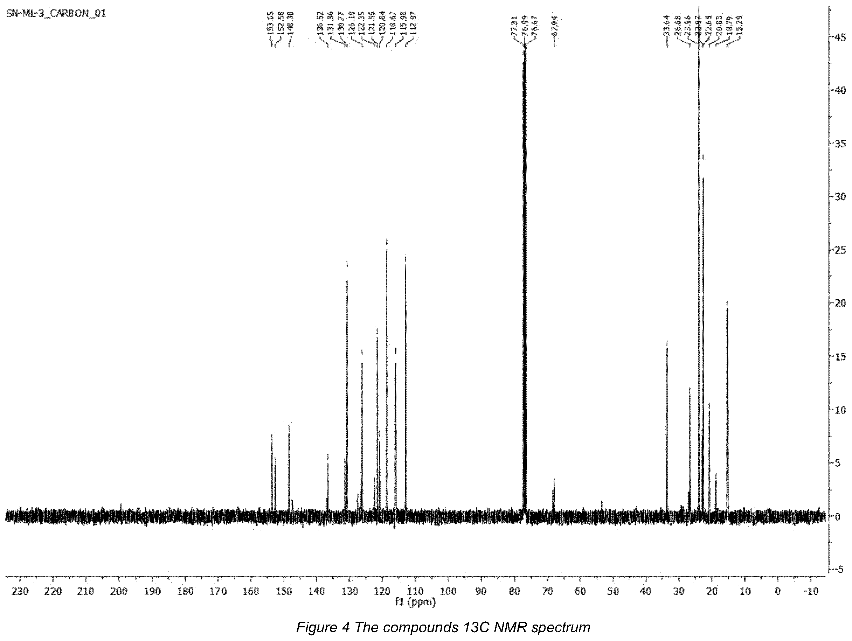

*Figure 4**: The compounds 13C NMR spectrum analysis, obtained after the synthesis of our novel compound.*

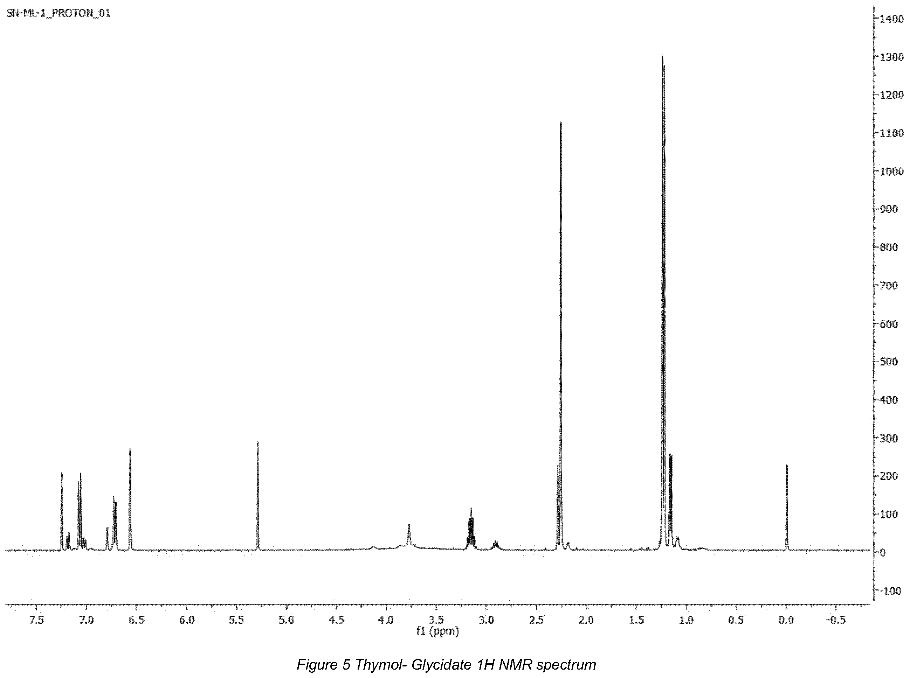

*Figure 5**: Thymol-Glycidate 1H NMR spectrum analysis, obtained during the synthesis of our novel compound.*

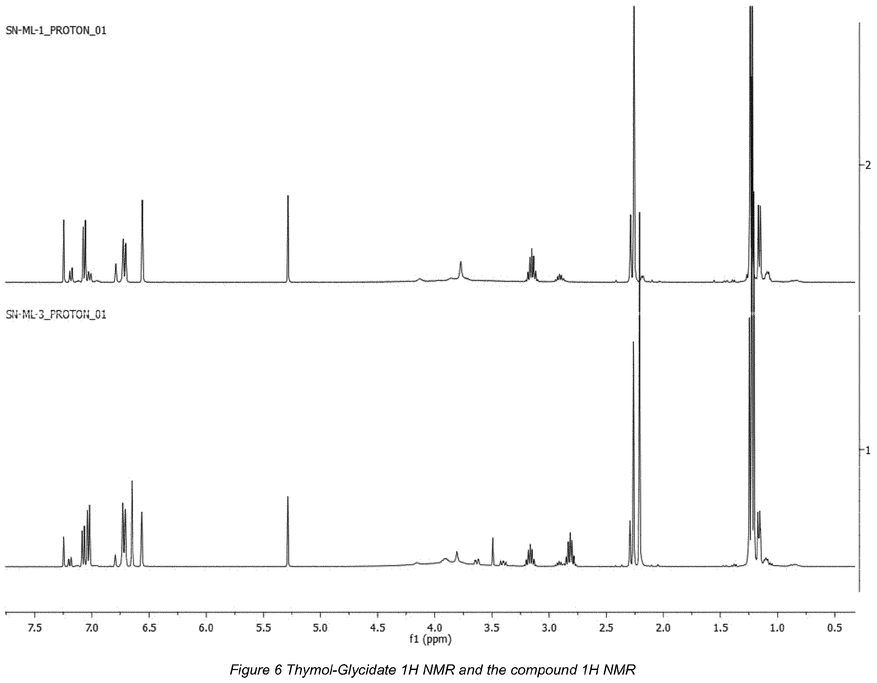

*Figure 6**: Thymol-Glycidate 1H NMR and the compound 1H NMR spectrum analysis, stacked, obtained during and after the synthesis of our novel compound.*

### Quality control

Our compound is light yellow to white solid substance that has a melting point of 68°C and a density of 1.09g mL⁻¹. For quantitative measurements as well as impurities exam, we use gass-mass or HPLC chromatography. By measuring the area of belonging peaks, we can know how much of our compound is in the mixture and what is its purity in comparison with the potential impurities. It is important to know what impurities are. In our case, impurities could be unreacted or hydrolized Thymol, Carvacrol or Glycine. Since we are dealing with a solvent of a low boiling point, dichloromethane, it easily evaporates during the purification step and does not pose a problem. Also, there can be traces of 4-dimethylamino pyridine (DMAP), dicyclohexyl carbodiimide (DCC), decomposition product of DCC - dicyclohexyl urea (DCU), di-tert-butyl dicarbonate (t-BOC)2O, tert-butyl alcohol (t-BuOH) and trifluoroacetic acid (TFA). After each reaction step of our synthesis, there is a work-up of reaction products, a purification step and final the separation on silica-gel column, after which we go to the next reaction.

### Method of novelty

The 1H NMR spectra shows that there is a small shift and perturbation in the signals of aromatic protons in the region of 6.5 to 7.5 ppm. That is because of the change in the orientation of those protons to the adjacent Carvacrol molecule which is connected to Thymol-Glycidate ester over the Nitrogen atom by an ionic bond. Because of the minor difference in the pKa value of the phenol group of Carvacrol (pKa=10.4) and Nitrogen atom of Thymol-Glycidate ester (pKa=9.60), there is an equilibrium of free Carvacrol and the ionic compound. The peak at 5.35ppm corroborates that statement. Based on that statement, it can be stated that the compound can easily dissociate into free Carvacrol and Thymol-glycidate ester. That ester can be further hydrolized in acidic media into Thymol and Glycine by a slow acid-catalyzed ester hydrolysis reaction. The obtained Thymol which is slowly released can be amgood source of constant new small amounts of Thymol after the fast Carvacrol release during the ionization step.

Carvacrol and Thymol are monoterpene phenols with chemical reactivity and properties of a phenol group. Their physiological activity is well known. The structure of the compound is selected to be synthesized because of a few factors and a higher effectiveness than sole Thymol and Carvacrol. The phenol groups of both Thymol and Carvacrol are very aggressive to pathogens: bacteria, fungi etc. These groups are needed for bioactivity of our product. What has been done was making the phenol group less reactive by reacting them with carboxylic and amino group of glycine. The product should be administered p.o. (per os). The novelty idea was to hydrolyze a part of the new molecule in the stomach by hydrochloric acid. Carvacrol and an ester of Glycine and Thymol will be slowly hydrolyzed into Thymol and Glycine. *In Vitro* studies of the compound have shown it having more effective and faster bactericide effect than existing Thymol and Carvacrol. The subject of this invention is to produce a potent entero-antiseptic for the treatment gastrointestinal infections in humans.

### Manufacturing of the compound

This text is a brief description of how the compound tablets have to be manufactured.

### Formula No. 1:

| | |
|---|---|
| Microcrystalline cellulose: | 488 mg |
| The novel compound: | 100 mg |
| Silicon dioxide: | 12 mg |
| TOTAL: | 600 mg |

This formula of the compound 600 mg tablets will be mixed and pressed using a regular tablet press. Such produced the compound tablets will be used for the treatment of gastroenteritis of the different ethiology.

### Formula No. 2:

| | |
|---|---|
| Microcrystalline cellulose: | 388 mg |
| The novel compound: | 100 mg |
| Coated cellulose: | 100 mg |
| Silicon dioxide: | 12 mg |
| TOTAL: | 600 mg |

This formula can be used for the treatment of Crohn's disease because the coated cellulose will allow the compound active compound to be released into the large intestine, particularly in the colon.

Both formulas should be taken with water or tea.

### Treatment of gastrointestinal infections in humans

- Case no. 1: I.S., Male, Age 47, USA, Los Angeles Symptoms: profuse diarrhea, bowel movement, light pain in the abdominal region.
- Case no. 2: J.M., Male, Age 68, USA, Santa Barbara Symptoms: profuse diarrhea, bowel movement, light pain in the abdominal region.
- Case no. 3: S.P., Male, Age 52, USA, Washington Symptoms: profuse diarrhea, bowel movement, light pain in the abdominal region.
- Case no. 4: V.A., Male, Age 39, Germany, Munich Symptoms: profuse diarrhea, bowel movement, light pain in the abdominal region.
- Case no. 5: L.N., Male, Age 56, Hungary, Budapest Symptoms: profuse diarrhea, bowel movement, light pain in the abdominal region.
- Case no. 6: S.A., Female, Age 38, USA, Sacramento Symptoms: profuse diarrhea, bowel movement, light pain in the abdominal region.
- Case no. 7: J.O., Female, Age 55, USA, Denver Symptoms: profuse diarrhea, bowel movement, light pain in the abdominal region.
- Case no. 8: N.W., Female, Age 59, USA, Boise Symptoms: profuse diarrhea, bowel movement, light pain in the abdominal region.
- Case no. 9: C.T. Female, Age 41, USA, Indianapolis Symptoms: profuse diarrhea, bowel movement, light pain in the abdominal region.
- Case no. 10: L.S. Female, Age 63, Austria, Vienna Symptoms: profuse diarrhea, bowel movement, light pain in the abdominal region.

### Additional information and results of testing

The reason for this testing was to check if the compound tablets, after a longer period of use, can cause any eventual side effects.

The test results were generated and monitored to obtain the relevant information and facts. In this study, the general monitored parameters were pulse, breathing, ECG (Electrocardiography), any discomfort, adverse events and/or allergy monitoring. These parameters were measured before the treatment with the compound and 15 minutes after its intake. The compound was ingested on the therapeutic days in each patient at 8.00 AM and 8.00 PM CET (paragraph [0026] and paragraph [0027]). After The compound had been consumed, the physician made observations for 30 min. Feeling of the patients during and after the intake of the compound will also be recorded for each participant.

As part of the biochemical analysis of patients' blood, the blood sample of each patient was checked for biochemical parameters. Blood samples were taken from each patient before the beginning of the test, on the 4th day from the tests beginning, and on the 8th day form the tests beginning. The last blood samples have to be checked 12 hours from the last intake of the compound. The biochemical parameters that were measured in blood samples include:
1. Liver status
2. C Reactive protein
3. Kidney Status

The dose was: 250 mg of the compound / 30 kg of the body weight every 12 hours for seven (7) days (paragraph [0026] and paragraph [0027]).

The clinical study started on November 5th, 2019, and was completed on November 29th, 2019.

List of participating male patients and schedule of administering the novel compound:

| No . | Pati ent | Gend er | A ge (y ea rs) | W eig ht (k g) | D ay 1 | | D ay 2 | | D ay 3 | | D ay 4 | | D ay 5 | | D ay 6 | | D ay 7 | | Dosage (mg) |
|---|---|---|---|---|---|---|---|---|---|---|---|---|---|---|---|---|---|---|---|
| | | | | | 8:0 0 AM | 8:0 0 PM | 8:0 0 AM | 8:0 0 PM | 8:0 0 AM | 8:0 0 PM | 8:0 0 AM | 8:0 0 PM | 8:0 0 AM | 8:0 0 PM | 8:0 0 AM | 8:0 0 PM | 8:0 0 AM | 8:0 0 PM | |
| 1 | D.S . | M | 2 5 | 6 3 | X | X | X | X | X | X | X | X | X | X | X | X | X | X | 525 |
| 2 | O.L . | M | 2 1 | 7 5 | X | X | X | X | X | X | X | X | X | X | X | X | X | X | 625 |
| 3 | B.P . | M | 3 7 | 6 8 | X | X | X | X | X | X | X | X | X | X | X | X | X | X | 567 |
| 4 | J.M . | M | 3 5 | 7 1 | X | X | X | X | X | X | X | X | X | X | X | X | X | X | 592 |
| 5 | I.N. | M | 2 8 | 7 2 | X | X | X | X | X | X | X | X | X | X | X | X | X | X | 600 |
| 6 | S.P . | M | 2 4 | 6 8 | X | X | X | X | X | X | X | X | X | X | X | X | X | X | 567 |
| 7 | G.J . | M | 3 6 | 7 8 | X | X | X | X | X | X | X | X | X | X | X | X | X | X | 650 |
| 8 | D.M . | M | 2 5 | 6 4 | X | X | X | X | X | X | X | X | X | X | X | X | X | X | 533 |
| 9 | I.M. | M | 2 2 | 6 6 | X | X | X | X | X | X | X | X | X | X | X | X | X | X | 550 |
| 10 | Z.U . | M | 3 1 | 8 1 | X | X | X | X | X | X | X | X | X | X | X | X | X | X | 675 |
| 11 | B.I. | M | 2 7 | 7 5 | X | X | X | X | X | X | X | X | X | X | X | X | X | X | 625 |
| 12 | M.S . | M | 3 8 | 6 9 | X | X | X | X | X | X | X | X | X | X | X | X | X | X | 575 |
| 13 | L.T. | M | 3 3 | 7 1 | X | X | X | X | X | X | X | X | X | X | X | X | X | X | 592 |
| 14 | A.V. | M | 2 7 | 6 5 | X | X | X | X | X | X | X | X | X | X | X | X | X | X | 542 |
| 15 | B.T . | M | 2 0 | 6 1 | X | X | X | X | X | X | X | X | X | X | X | X | X | X | 508 |
| 16 | V.S . | M | 3 9 | 8 4 | X | X | X | X | X | X | X | X | X | X | X | X | X | X | 700 |
| 17 | M.M . | M | 2 5 | 7 3 | X | X | X | X | X | X | X | X | X | X | X | X | X | X | 608 |
| 18 | I.G. | M | 2 9 | 7 1 | X | X | X | X | X | X | X | X | X | X | X | X | X | X | 592 |
| 19 | N.B . | M | 3 4 | 8 3 | X | X | X | X | X | X | X | X | X | X | X | X | X | X | 692 |
| 20 | T.N . | M | 3 3 | 6 8 | X | X | X | X | X | X | X | X | X | X | X | X | X | X | 567 |
| 21 | P.P . | M | 2 1 | 7 4 | X | X | X | X | X | X | X | X | X | X | X | X | X | X | 617 |
| 22 | M.A . | M | 2 6 | 6 6 | X | X | X | X | X | X | X | X | X | X | X | X | X | X | 550 |
| 23 | J.L. | M | 2 4 | 7 8 | X | X | X | X | X | X | X | X | X | X | X | X | X | X | 650 |
| 24 | I.S. | M | 3 8 | 7 4 | X | X | X | X | X | X | X | X | X | X | X | X | X | X | 617 |
| 25 | Z.R . | M | 3 1 | 6 6 | X | X | X | X | X | X | X | X | X | X | X | X | X | X | 550 |
| 26 | T.S . | M | 2 8 | 6 9 | X | X | X | X | X | X | X | X | X | X | X | X | X | X | 575 |
| 27 | I.O. | M | 2 2 | 6 3 | X | X | X | X | X | X | X | X | X | X | X | X | X | X | 525 |
| 28 | P.V . | M | 2 7 | 7 1 | X | X | X | X | X | X | X | X | X | X | X | X | X | X | 592 |
| 29 | N.R . | M | 3 4 | 8 0 | X | X | X | X | X | X | X | X | X | X | X | X | X | X | 667 |
| 30 | Z.T. | M | 3 7 | 6 9 | X | X | X | X | X | X | X | X | X | X | X | X | X | X | 575 |

List of participating female patients and schedule of administering the novel compound:

| No. | Pa tie nt | Ge nd er | Age(years) | W eig ht (k g) | D ay 1 | | D ay 2 | | D ay 3 | | D ay 4 | | D ay 5 | | D ay 6 | | D ay 7 | | Do sa ge ( m g) |
|---|---|---|---|---|---|---|---|---|---|---|---|---|---|---|---|---|---|---|---|
| | | | | | 8:0 0 AM | 8:0 0 PM | 8:0 0 AM | 8:0 0 PM | 8:0 0 AM | 8:0 0 PM | 8:0 0 AM | 8:0 0 PM | 8:0 0 AM | 8:0 0 PM | 8:0 0 AM | 8:0 0 PM | 8:0 0 AM | 8:0 0 PM | |
| 1 | A. S. | F | 2 1 | 5 0 | X | X | X | X | X | X | X | X | X | X | X | X | X | X | 4 2 0 |
| 2 | T. T. | F | 2 2 | 6 0 | X | X | X | X | X | X | X | X | X | X | X | X | X | X | 5 0 0 |
| 3 | S. D. | F | 2 2 | 5 4 | X | X | X | X | X | X | X | X | X | X | X | X | X | X | 4 5 3 |
| 4 | M. P. | F | 3 6 | 5 6 | X | X | X | X | X | X | X | X | X | X | X | X | X | X | 4 7 3 |
| 5 | I. J. | F | 2 4 | 5 7 | X | X | X | X | X | X | X | X | X | X | X | X | X | X | 4 8 0 |
| 6 | M. M. | F | 2 5 | 5 4 | X | X | X | X | X | X | X | X | X | X | X | X | X | X | 4 5 3 |
| 7 | I. S. | F | 3 1 | 6 2 | X | X | X | X | X | X | X | X | X | X | X | X | X | X | 5 2 0 |
| 8 | LJ .V. | F | 2 3 | 5 1 | X | X | X | X | X | X | X | X | X | X | X | X | X | X | 4 2 7 |
| 9 | S. P. | F | 2 8 | 5 2 | X | X | X | X | X | X | X | X | X | X | X | X | X | X | 4 4 0 |
| 10 | A. B. | F | 2 1 | 6 4 | X | X | X | X | X | X | X | X | X | X | X | X | X | X | 5 4 0 |
| 11 | M. K. | F | 2 7 | 6 0 | X | X | X | X | X | X | X | X | X | X | X | X | X | X | 5 0 0 |
| 12 | A. V. | F | 2 2 | 5 5 | X | X | X | X | X | X | X | X | X | X | X | X | X | X | 4 6 0 |
| 13 | K. M. | F | 3 5 | 5 6 | X | X | X | X | X | X | X | X | X | X | X | X | X | X | 4 7 3 |
| 14 | J. M. | F | 2 8 | 5 2 | X | X | X | X | X | X | X | X | X | X | X | X | X | X | 4 3 3 |
| 15 | S. C. | F | 2 9 | 4 8 | X | X | X | X | X | X | X | X | X | X | X | X | X | X | 4 0 7 |
| 16 | A. P. | F | 2 1 | 6 7 | X | X | X | X | X | X | X | X | X | X | X | X | X | X | 5 6 0 |
| 17 | A. D. | F | 2 0 | 5 8 | X | X | X | X | X | X | X | X | X | X | X | X | X | X | 4 8 7 |
| 18 | V. M. | F | 3 3 | 5 6 | X | X | X | X | X | X | X | X | X | X | X | X | X | X | 4 7 3 |
| 19 | J. D. | F | 3 6 | 6 6 | X | X | X | X | X | X | X | X | X | X | X | X | X | X | 5 5 3 |
| 20 | M. D. | F | 3 8 | 5 4 | X | X | X | X | X | X | X | X | X | X | X | X | X | X | 4 5 3 |
| 21 | J. D. | F | 2 6 | 5 9 | X | X | X | X | X | X | X | X | X | X | X | X | X | X | 4 9 3 |
| 22 | S. D. | F | 2 2 | 5 2 | X | X | X | X | X | X | X | X | X | X | X | X | X | X | 4 4 0 |
| 23 | K. P. | F | 3 4 | 6 2 | X | X | X | X | X | X | X | X | X | X | X | X | X | X | 5 2 0 |
| 24 | J. K. | F | 3 8 | 5 9 | X | X | X | X | X | X | X | X | X | X | X | X | X | X | 4 9 3 |
| 25 | I. S. | F | 2 6 | 5 2 | X | X | X | X | X | X | X | X | X | X | X | X | X | X | 4 4 0 |
| 26 | M. L. | F | 2 4 | 5 5 | X | X | X | X | X | X | X | X | X | X | X | X | X | X | 4 6 0 |
| 27 | A. R. | F | 3 1 | 5 0 | X | X | X | X | X | X | X | X | X | X | X | X | X | X | 4 2 0 |
| 28 | Z. A. | F | 2 2 | 5 6 | X | X | X | X | X | X | X | X | X | X | X | X | X | X | 4 7 3 |
| 29 | T. P. | F | 3 1 | 6 4 | X | X | X | X | X | X | X | X | X | X | X | X | X | X | 5 3 3 |

Based on the results obtained during this clinical pilot study of the compound on 60 humans, the following conclusions can be made:
1. The participants of the study showed tolerance to the compound upon the prescribed dosage.
2. There were no symptoms of allergy due to the prescribed dosage of the compound.
3. During the intake of the compound, pulse frequency and blood pressure were not significantly altered.
4. Electrocardiography measurements during the intake of the compound were regular without any pathological and irregular oscillations.
5. Respiratory function was also monitored during the clinical study. The respiratory function of patients was not disturbed, and none of the patients reported any irregularity in respiratory function during the study.
6. No irregular parameters were reported in the liver function biochemical analysis from blood samples during the study. Therefore, the compound did not have any negative clinical or acute effects on the hepatic function of the participants volunteering in this study.
7. No irregular parameters were reported during the testing of kidney functions for the period of testing the compound. The test results of both urine and blood parameters related to kidney function, have suggested that the compound did not have any negative effects on the kidneys of the patients.
8. The results of the C Reactive Protein (CRP) measurements during the study with the compound suggest that no inflammation was developed.

### The compound in vitro test

Here are the findings of the comparative examination of selected pathogens against the compound and the antibiotics Cephalosporin and Ampicillin:

| Bacteria | Method | The novel compo und | R | Cephalosp orin | R | Ampicilli n | R |
|---|---|---|---|---|---|---|---|
| Staphylococcus aureus | (Müller)Hinton-Agar | 32 mm | E S | 19 mm | VS | 0 mm | R |
| Escherichia coli 0157H5 | (Müller)Hinton-Agar | 29 mm | E S | 18 mm | VS | 7 mm | M S |
| Salmonella typhimurium DT104 | (Müller)Hinton-Agar | 30 mm | E S | 19 mm | VS | 6 mm | M S |
| Streptococcus pneumonia | (Müller)Hinton-Agar | 36 mm | E S | 20 mm | VS | 0 mm | R |
| Streptococcus pyogenes | (Müller)Hinton-Agar | 34 mm | E S | 17 mm | VS | 3 mm | R |
| Streptococcus uberis | (Müller)Hinton-Agar | 37 mm | E S | 21 mm | ES | 0 mm | R |
| Listeria monocytogenes | (Müller)Hinton-Agar | 38 mm | E S | 24 mm | ES | 5 mm | M S |
| E. coli | (Müller)Hinton-Agar | 32 mm | E S | 19 mm | VS | 2 mm | R |
| Salmonella | (Müller)Hinton-Agar | 34 mm | E S | 21 mm | ES | 8 mm | M S |
| Campylobacter | (Müller)Hinton-Agar | 35 mm | E S | 16 mm | VS | 0 mm | R |
| Shigella | (Müller)Hinton-Agar | 31 mm | E S | 18 mm | VS | 6 mm | M S |
| Vibrio | (Müller)Hinton-Agar | 33 mm | E S | 23 mm | ES | 4 mm | R |
| Yersinia | (Müller)Hinton-Agar | 30 mm | E S | 20 mm | VS | 1 mm | R |
| Listeria | (Müller)Hinton-Agar | 32 mm | E S | 21 mm | ES | 2 mm | R |

Amount used: The novel compound 0.05 mL; Cephalosporin 0.1 mL; Ampicillin 0.2 mL.

Evaluation of the results by zone diameter:
0mm - 4mm: resistant: R
5 - 9 mm: moderately sensitive: MS
10 - 15 mm : sensitive: S
16 - 20 mm : very sensitive: VS
21 mm and more: extremely sensitive: ES

### Testing of the toxicity of the compound in tablet form

The subject of the testing was to determine the acute toxicity - LD50 of the compound in tablet form on mice and rats. Both mice and rats were specially selected for the testing of the toxicity of the compounds, medicine and chemicals. Only the acute toxicity of the testing was done since the active therapy with the compound takes from one (1) - three (3) days. Sub-acute toxicity was not necessary and applicable since long treatment with (the compound) will not be in function or planned. Testing was done in the oral application (per os - p.o.) in both white lab rats and white lab mice. Parenteral application was done by intraperitoneal (i.p.) application in both lab animals: white mice and white rats. Groups of the 100 lab mice and rats were place into glass compartments. They were regularly consuming the food manufactured for these lab animals. Water consumption was always available. References for this testing was Carvacrol 99,5% concentration for rats LD50 810 mg / kg oral (p.o.) and Thymol 99,8% concentration rats with LD50 192 mg / kg (par enteral i.p.). The compound is a compound manufactured by the novel patented process of manufacturing by bouncing Carvacrol 50% and Thymol 50%. In this toxicology test - acute toxicity of the compound were tested:
Oral LD50 p.o. - mice
Oral LD50 p.o. - rats
Parenteral - intraperitoneal LD50 i.p. - mice
Parenteral - intraperitoneal LD50 i.p. - rats

### Oral application of the compound - LD50 - testing and doses

The oral acute toxicity LD50 testing was upon the standard procedure: application p.o. with the special lab pipette easy to administer liquid product: orally to the lab animals.

### Results of the oral testing of LD50 of the compound on mice

Oral (p.o.) application of the compound testing LD50 has been by mice: 1 mg/kg, 5 mg/kg, 10 mg/kg, 20 mg/kg, 50 mg/kg, 100 mg/kg, 150 mg/kg, 200 mg/kg, 300 mg/kg, 400 mg/kg, 500 mg/kg, 600 mg/kg, 650 mg/kg, 750 mg/kg, 850 mg/kg, 1,000 mg/kg, 1250mg/kg, 1,500 mg/kg, 1,750 mg/kg and 2,000 mg/kg. At the level of oral dose LD50 2,000 mg/kg - twenty- one (21) mice out of a hundred (100), have changed their behavior and demonstrated light discomfort: running as well as anxiety. Breathing and cardiac functions were regular, function of the thorax was regular and without visual changes. Discomfort symptoms were noticed after the applications, they disappeared after 1- 3 min., diverse from mouse to mouse. No mouse has ended lethally during this test. Conclusion based on these test results was: The compound in pill form has an oral LD50 over 2,000 mg/kg for mice (p.o.). The compound is low toxic in the applied doses for mice orally (p.o.).

### Results of the oral testing of LD50 of The compound on rats

Oral (p.o.) application of the compound testing LD50 has been on rats: 1 mg/kg, 5 mg/kg, 10 mg/kg, 20 mg/kg, 50 mg/kg, 100 mg/kg, 150 mg/kg, 200 mg/kg, 300 mg/kg, 400 mg/kg, 500 mg/kg, 600 mg/kg, 650 mg/kg, 750 mg/kg, 850 mg/kg, 1,000 mg/kg, 1250 mg/kg, 1,500 mg/kg, 2,000 mg/kg, 2,250 mg/kg and 2,500 mg/kg. At the level of oral dose LD50 2,500 mg/kg - thirty-seven (37) rats out of a hundred (100), have changed their behavior and demonstrated light discomfort: running over the glass cubical, as well as anxiety. Breathing and cardiac functions were regular, function of the thorax was regular and without visual changes. Discomfort symptoms were noticed after the applications, they disappeared after 1 - 3 min., diverse from rat to rat. No rats have ended lethally during this test. Conclusion based on these test results was: the compound in tablet form has an oral LD50 over 2,500 mg/kg for rats (p.o.). The compound is low toxic in the applied doses for rats orally (p.o.).

### Parenteral application of the compound - LD50 - testing and doses

Parenteral-intraperitoneal testing of the compound LD50 i.e. by pharmaceutical lab needle and syringe, designed for the easy application to the lab animals intraperitoneally.

### Results of the parenteral - intraperitoneal (i.p) testing on mice

Intraperitoneal application (i.p.) of the compound tablet, partly diluted into a glucose. The testing LD50 has been done on mice: 1 mg/kg, 5 mg/kg, 10 mg/kg, 20 mg/kg, 50 mg/kg, 100 mg/kg, 150 mg/kg, 200 mg/kg, 300 mg/kg, 400 mg/kg, 500 mg/kg, 600 mg/kg, 650 mg/kg, 750 mg/kg, 850 mg/kg, 1,000 mg/kg, 1250 mg/kg, 1,500 mg/kg, 1,750 mg/kg, 2,000 mg/kg, and 2,250 mg/kg. At the level of i.p. dose LD50 2,250 mg/kg - twenty-seven (27) mice out of a hundred (100), have changed their behavior and demonstrated discomfort: running as well as anxiety. Breathing and cardiac functions were regular, function of the thorax was regular and without visual changes. Discomfort symptoms were noticed after the applications, they disappeared after 3 - 5 min., diverse from mouse to mouse. No mouse has ended lethally during this test. Conclusion based on these test results was: The compound in tablet form has parenteral - intraperitoneal LD50 over 2,250 mg/kg for mice (i.p). The compound is low toxic in the applied doses for mice parenterally - intraperitoneally (i.p.).

### Results of the parenteral - intraperitoneal (i.p) testing on rats

Intraperitoneal application (i.p.) of the compound tablet, partly diluted into a glucose. The testing LD50 has been done on rats: 1 mg/kg, 5 mg/kg, 10 mg/kg, 20 mg/kg, 50 mg/kg, 100 mg/kg, 150 mg/kg, 200 mg/kg, 300 mg/kg, 400 mg/kg, 500 mg/kg, 600 mg/kg, 650 mg/kg, 750 mg/kg, 850 mg/kg, 1,000 mg/kg, 1250 mg/kg, 1,500 mg/kg, 1,750 mg/kg, 2,000 mg/kg, 2,250 mg/kg, and 2,500 mg/kg. At the level of i.p. dose LD50 2,500 mg/kg - thirty-five (35) rats out of a hundred (100), have changed their behavior and demonstrated discomfort: running over the glass cubical as well as anxiety. Breathing and cardiac functions were regular, functions of the thorax were regular and without visual changes. Discomfort symptoms were noticed after the applications, they disappeared after 5 - 6 min., diverse from the rat to rat. No rat has ended lethally during this test. Conclusion based on these test results was: The compound injectable solution has parenteral - intraperitoneal LD50 over 2,500 mg/kg for rat (i.p.). The compound is low toxic in the applied doses for rats parenterally - intraperitoneally (i.p.).

### Mutagenic and cancer-genic tests of the compound tablet, partly diluted into a glucose injectable solution

(The subject of the testing was to determine the mutagenic and carcinogenic effect of the compound in tablet form). The test has been done by the Ames test. This test which is a widely and often used method that use pathogens in most cases bacteria's in order to test whether a given chemical, compound and/or medicine can cause any damage to the DNA of the test organism. This method is fast to conduct and easy to follow to check mutagenic or carcinogenic effect of the tested compound. In the lab dish with a the specially selected Salmonella strain which requires histidine, was added to a rat liver extract. The test has been done using the control-experimental method. In the dish volume of the 20 mL of liquid considered to be a control one, had no the compound in it. Into the dish we consider to be experimental one with also a volume of 20 mL, we added 2 mL of the compound, partly diluted into a glucose injectable solution. The mutagenic test results were negative in both lab dishes - control and experimental plates. The results were identical with no signs of the mutations. Based on this preliminary result, conclusion could be made that the compound in tablet form possess no mutagenic effects. Since the mutation is often linked to cancer, these results suggest that the compound in tablet form has no carcinogenic effect.

In the pilot test at the same Institute, the effect of the compound on the mammalian cells, mitotic division has also been tested. It has been investigated if the compound in tablet form will have any negative - suppressive effect on the mitotic cells division. After the observation of the different levels of the mitotic cells division and activities with a presence of the compound, no negative effects of it were diagnosed. It is a positive sign that the compound in tablet form might be used for on pregnant mammals, without danger of the teratogenic effect on the fetus in the wombs, by the pregnant mammals.

## Claims

1. A compound manufactured with the procedure of synthesis of bouncing Thymol and Carvacrol into one solid and stabile compound having a structure as shown in Formula:

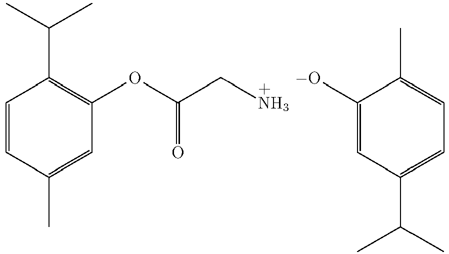

2. The compound according to claim 1, as an active compound/potent entero-antiseptic for use in the treatment of infectious acute gastroenteritis in humans, caused by *E. coli, Salmonella, Campylobacter, Shigella, Vibrio, Yersinia, Listeria.*

3. The compound according to claim 1, in its second formulation, with coated cellulose, as an active compound/potent entero-antiseptic for use in the treatment of chronic intestinal infections: Crohn's disease (inflammatory bowel disease - IBD).

## Patentansprüche

1. Eine Verbindung, hergestellt durch Synthese von Thymol und Carvacrol zu einer festen und stabilen Verbindung mit der in Formel I dargestellten Struktur:

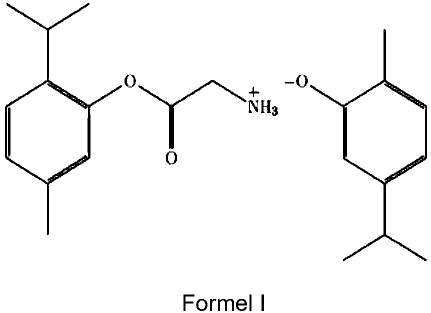

2. Die Verbindung gemäß Patentanspruch 1 als Wirkstoff/potentes Enteroantiseptikum zur Anwendung bei der Behandlung von akuter infektiöser Gastroenteritis beim Menschen, verursacht durch E. Coli, Salmonella, Campylobacter, Shigella, Vibrio, Yersinia, Listeria.

3. Die Verbindung gemäß Patentansprüchen 1, in ihrer zweiten Formulierung mit beschichteter Cellulose als Wirkstoff/potentes Enteroantiseptikum zur Anwendung bei der Behandlung vonchronischer Darminfektionen: Morbus Crohn (entzündliche Darmerkrankung - CED).

## Revendications

1. Composé fabriqué selon le procédé de synthèse consistant à combiner du thymol et du carvacrol en un composé solide et stable présentant la structure représentée dans la formule I :

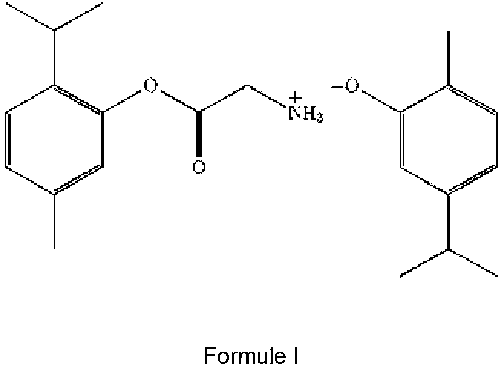

2. Le composé selon la revendication 1, en tant que composé actif/entéro-aniseptique puissant pour être utilisé dans le traitement des gastro-entérites infectieuses aiguës chez l'homme causées par Escherichia coli, Salmonella, Campylobacter, Shigella, Vibrio, Yersinia, Listeria.

3. Le composé selon les revendications 1 dans sa deuxième formulation avec de la cellulose enrobée comme ingrédient actif/entéroantisectique puissant pour une utilisation dans le traitement des infections intestinales chroniques: maladie de Crohn (maladie inflammatoire de l'intestin - MII).
